# DEMANDE DE BREVET EUROPEEN

(11) **EP 1 431 401 A2**
(43) Date de publication de la demande: **23.06.2004**
(21) Numéro de dépôt: 04290402.9
(22) Date de dépôt: 08.12.1999
(51) Int. Cl.: C12Q 1/68, C12Q 1/70, C12Q 1/00

(54) **Méthode de détection et/ou de quantification d'une fonction connue à partir d'un échantillon d'acides nucléiques**

(30) Priorité: 08.12.1998 FR 9815487
(62) Demande divisionnaire de: 99957373.6
(71) Demandeur: Proteus, 30000 Nîmes (FR)
(72) Inventeur: BLOCH, Jean-François, F-30000 Nîmes (FR); DUPRET, Daniel, F-30420 Calvisson (FR); MASSON, Jean-Michel, F-31400 Toulouse (FR); LEFEVRE, Fabrice, 30900 Nîmes (FR); SCHERRER, Sandrine, 30900 Nîmes (FR)
(74) Mandataire: Breese, Pierre

(57) **Abrégé**

La présente invention concerne une méthode de détection d'une fonction connue, à partir des acides nucléiques présents dans un échantillon, caractérisée en ce qu'elle comprend les étapes suivantes (a) la préparation, à partir des acides nucléiques de l'échantillon, de molécules d'acides nucléiques comprenant le ou les gènes codant pour la ou les protéines correspondant à ladite fonction, et les éléments de contrôle nécessaires à la transcription et à la traduction du ou desdits gènes; (b) la transcription et la traduction in vitro des molécules d'acide nucléique préparée(s) à l'étape (a); (c) la détection et/ou la mesure de la fonction correspondant à la ou aux protéines produites à l'étape (b).

## Description

La présente invention a pour objet une méthode de détection et/ou de quantification d'une fonction connue à partir d'un échantillon d'acides nucléiques. La méthode de l'invention trouve notamment son application dans l'analyse in vitro d'une fonction connue à partir d'un échantillon d'acides nucléiques. Cette méthode trouve aussi son application dans le domaine du diagnostic, où la fonction recherchée est caractérisée par exemple par la présence d'une activité enzymatique, de cellules cancéreuses, d'organismes pathogènes (bactériens ou viraux), d'une mutation spécifique, d'un gène étranger.

Aujourd'hui, la recherche de séquences polynucléotidiques cibles représente un objectif majeur dans de nombreux laboratoires de recherche impliqués dans de nombreux domaines d'activité, et principalement dans le domaine médical ou agroalimentaire ou l'industrie chimique. Dans ces domaines, la recherche de séquences cibles visent par exemple :
- Le diagnostic de virus à l'origine de maladies, telles que le Sida (HIV) ou l'hépatite B (HBV).
- Le diagnostic spécifique de maladies d'origine bactérienne, telle que la tuberculose ou la lèpre.
- Le diagnostic de mutations à l'origine de maladies génétiques ou de cancers cellulaires.
- Le diagnostic d'une contamination bactérienne dans une chaîne agroalimentaire.
- La recherche des microorganismes impliqués dans la corrosion biologique des canalisations ou des containers utilisés dans des procédés industriels.

La difficulté majeure des méthodes de diagnostic utilisées dans l'art antérieur réside dans la spécificité, la sensibilité, la rapidité et la reproductibilité du test de diagnostic utilisé. Il est indispensable de pouvoir donner un résultat certain. Il convient donc de disposer d'une méthode pouvant être réalisée à partir d'un échantillon de départ de quelque nature ou origine qu'il soit. Il faut en outre une méthode permettant de réduire les faux positifs dus à la composition de l'échantillon ou générés par la méthode mise en oeuvre.

Or, comme indiquées précédemment, les méthodes de l'art antérieur sont essentiellement basées sur la détection de fragments d'acides nucléiques permettant éventuellement de quantifier un gène. Parmi celles-ci, on peut citer :
- Les tests utilisant des sondes génétiques, où la quantité de séquence cible détectée dépend de la taille et de l'homologie de la sonde. Ces tests sont appliqués à la détection de différents organismes, comme des bactéries, des virus, des mycobactéries, des champignons ou d'autres parasites. Le problème de ces tests réside dans leur faible sensibilité.
- Les tests d'amplification type PCR ou autres qui permettent de détecter spécifiquement une information génétique avec une réelle sensibilité, qui doit être tout de même nuancée par des problèmes techniques conduisant à des faux positifs.

Lors d'un diagnostic de séquences par amplification PCR, on n'est pas sûr de la nature de l'amplicon, et dans le cas où la protéine analysée est recherchée par immunodétection, les résultats peuvent correspondre à une réaction croisée non spécifique. De plus dans ces deux cas, on ne connaît pas l'activité de la protéine recherchée.

De plus, ces tests de détection ne permettent pas d'obtenir des informations sur l'activité fonctionnelle du produit du gène, ce qui peut :
- engendrer des faux positifs lors de la détection de séquences homologues à la place de la séquence cible,
- engendrer des erreurs de diagnostique, comme dans le cas de la détection d'un gène muté qui code pour une protéine non fonctionnelle,
- aboutir à un niveau d'information insuffisant, comme par exemple la détection d'un pathogène sans pour autant donner d'indication sur sa virulence ou sa sensibilité à un traitement, ce qui conduit à un défaut de caractérisation,
- aboutir à un seuil de sensibilité trop faible.

On peut aussi citer les tests immunologiques ou d'incorporation de radioactivité lors de synthèse de protéines qui permettent de détecter et éventuellement de quantifier directement une protéine. Des anticorps sont en effet utilisés en routine pour identifier spécifiquement un antigène dans un échantillon. Cependant, ces tests ne donnent généralement pas d'information sur l'activité fonctionnelle de la protéine.

Il existe également dans l'art antérieur, des tests permettant d'effectuer directement la détection d'une fonction à partir d'un échantillon sans passer par les acides nucléiques présents dans ledit échantillon. Il s'agit alors de travailler directement sur l'ensemble des facteurs présents dans l'échantillon et susceptibles d'agir sur le test fonctionnel. Cependant, il ne sera pas possible de distinguer une même fonction provenant de deux facteurs différents. Il peut s'agir par exemple d'une fonction associée à deux virus différents qu'il ne sera pas possible de distinguer avec le même test fonctionnel. On peut citer, la détection de l'activité fonctionnelle reverse transcriptase proposée dans la demande de brevet PCT publiée sous le No. WO96/23076, dont on ne peut pas déterminer si l'activité reverse transcriptase provient d'HIV ou d'un autre virus.

On peut enfin citer les tests de détection de fonction et de variants fonctionnels basés sur des étapes réalisées *in vivo* comme les antibiogrammes, ou des techniques d'essais phénotypiques par exemple sur le virus HIV. Si les souches manipulées sont dangereuses, elles nécessitent des conditions de sécurité particulières donc des aménagements spéciaux des laboratoires. Outre leur complexité de mise en oeuvre qui les rend difficilement automatisable, ces tests sont onéreux et de mise en oeuvre longue et fastidieuse. Ainsi, dans le cas des virus, l'isolement des virus dure de 4 à 10 semaines. Des tests plus récents fondés sur des méthodes de clonage d'une activité enzymatique prennent encore de 3 à 4 semaines. De plus, ces tests ne permettent pas toujours de détecter les variants minoritaires.

Le but de la présente invention est précisément d'offrir une méthode de détection et avantageusement de quantification d'une fonction connue éventuellement présente dans un échantillon ne présentant pas les inconvénients ci-dessus. La méthode de l'invention s'applique, bien entendu, à la détection et/ou la quantification simultanée ou non d'une ou plusieurs fonctions connues présentes dans le même échantillon ou dans des échantillons différents regroupés. Ainsi, l'emploi par la suite du terme "fonction" au singulier couvrira également le terme "fonctions" au pluriel et vice versa, sauf lorsqu'il sera indiqué explicitement qu'il s'agit de la mise en oeuvre de la méthode de l'invention sur plusieurs fonctions. La méthode de l'invention permet donc non seulement de détecter et avantageusement de quantifier une ou plusieurs fonctions, mais aussi de les caractériser d'un point de vue biochimique à partir d'un seul échantillon.

On entend par fonction, l'activité d'une ou plusieurs protéines spécifiques d'au moins un organisme ou d'au moins un processus, et que l'on détecte et/ou quantifie selon la présente invention par le biais d'un test de la fonction de la ou desdites protéines. Dans le cadre de cette invention, le phénotype est considéré comme une fonction. La méthode de l'invention trouve donc tout particulièrement son application dans le domaine du diagnostic in vitro et notamment en matière d'analyse d'une fonction. En effet, on entend aussi par fonction, au sens de la présente invention, la manifestation d'une propriété observable correspondant à l'expression d'un ou plusieurs gènes. Cette manifestation peut se traduire par l'expression d'un trait morphologique, d'un syndrome clinique, d'une résistance à un composé toxique, de l'activité d'une protéine ou encore de la production d'une substance comme un métabolite, un antibiotique, etc... . Dans le cadre de cette définition de la fonction, l'invention permet aussi l'analyse in vitro d'une fonction correspondant au résultat de l'expression de plusieurs gènes, les protéines correspondantes étant toutes nécessaires à l'expression de ladite fonction. Dans cette forme de réalisation de l'invention, les gènes codant pour cette ou ces fonctions peuvent être situés :
- sur le même fragment d'ADN, dans le cas d'un phénotype exprimé par un opéron,
- à différents endroits de l'ADN génomique, dans le cas de certaines voies métaboliques.

Le phénotype analysé selon le procédé de l'invention peut donc ne pas être seulement celui associé à une protéine, mais celui associé à un ensemble de protéines. Il s'agit par exemple, comme indiqué précédemment, d'un phénotype correspondant à la synthèse d'un métabolite (métabolite énergétique, antibiotique, etc...), à la dégradation d'un composé (de type nutriment ou xénobiotique) ou à l'assemblage d'une protéine complexe constituée de plusieurs sous-unités identiques ou différentes.

La fonction peut correspondre par exemple à une activité enzymatique ou une affinité. Dans le cadre de la mise en évidence d'une activité enzymatique, tout type de substrat spécifique peut être envisagé par l'homme de métier pour mettre en évidence la présence de la fonction recherchée. L'homme de métier pourra par exemple se référer à des ouvrages comme Methods In Enzymology ou Annual Review Of Biochemistry, dans lesquelles un grand nombre de méthodes de dosage d'enzymes et de préparation de substrats ont été décrites. Dans le cas de la mise en évidence d'une affinité par exemple d'un antigène pour un anticorps, d'une protéine pour de l'ADN, d'un récepteur pour un ligand etc... . la mise en évidence d'une fonction peut être réalisée par exemple par des tests tels que la fixation de ligands marqués par un isotope ou par une enzyme ou par un fluorophore, par une détection immunologique utilisant des anticorps marqués par un métal ou par une enzyme ou par un fluorophore.

On entend par organisme, tout type d'organisme ou micro-organisme, comme des virus, des bactéries, des algues, des champignons, ou tout produit comportant des acides nucléiques synthétiques ou naturel permettant l'expression des fonctions recherchées par le procédé de l'invention.

On entend par processus, le développement d'une maladie, d'une infection ou de cellules par exemple cancéreuses ou de contaminants. Ces processus peuvent avoir lieu sur un hôte ou dans un procédé industriel.

On entend par extrait de traduction un extrait comportant tous les facteurs nécessaires à l'étape de traduction à partir des messagers néosynthétisés comme par exemple des ribosomes, des tRNA, des facteurs d'élongation, des facteurs d'initiation etc... .

On entend par fonction connue, la fonction qui est analysée selon le procédé de l'invention et qui peut être détectée et/ou mesurée par un test fonctionnel.

Le matériel biologique à partir duquel est obtenu l'échantillon sur lequel est réalisé la méthode de l'invention peut être tout échantillon susceptible de contenir des acides nucléiques de type humain, animal, végétal, microbien, viral ou des échantillons provenant du sol, de l'eau, de biopsie, de cellules diverses, un processus ou un organisme. Ces échantillons peuvent aussi correspondre à des produits de toute méthode d'amplification de l'ADN génomique, de l'ADN synthétique, de l'ARN, ou tout produit d'acides nucléiques issus de traitements couramment utilisés par l'homme de métier. Il peut s'agir, bien entendu, d'un échantillon biologique brut comme du sang, des tissus, de l'urine ou tout autre liquide corporel comme le liquide cérébrospinal, synovial, pleural, péricardial ou préalablement traité pour préparer les acides nucléiques qu'il contient.

La présente invention a donc pour objet une méthode de détection et/ou de quantification d'une fonction connue, à partir des acides nucléiques présents dans ledit échantillon, caractérisée en ce qu'elle comprend les étapes suivantes :
a) la préparation, à partir des acides nucléiques de l'échantillon, de molécules d'acides nucléiques comprenant le ou les gènes codant pour la ou les protéines correspondant à ladite fonction, et les éléments de contrôle nécessaires à la transcription et à la traduction du ou desdits gènes;
b) la transcription et la traduction *in vitro* des molécules d'acide nucléique préparée(s) à l'étape (a);
c) la détection et/ou la mesure de la fonction correspondant à la ou aux protéines produites à l'étape (b) .

Lorsque le procédé de l'invention est une méthode de détection, on prépare à l'étape (a) au moins une molécule d'acide nucléique comprenant le ou les gènes codant pour la ou les protéines correspondant à ladite fonction. Tout préférentiellement, lorsque la méthode de l'invention est une méthode de quantification, on prépare à l'étape (a), une quantité de molécules d'acides nucléiques proportionnelle à la quantité du ou desdits gènes éventuellement présent(s) dans les acides nucléiques de l'échantillon. En conséquence, les procédés mis en oeuvre à l'étape (a) comme décrit ci-après, sont mis en oeuvre de façon à assurer cette proportionnalité.

Afin de faciliter l'exposé de l'invention, on désigne à l'étape (a) de la méthode de l'invention, sous le terme de gène, toute séquence d'acides nucléiques permettant l'expression de la ou des protéine(s) correspondant à la fonction détectée. Il peut donc s'agir de séquence d'ADN ou d'ARN.

La détection et/ou la mesure de la fonction correspondant à la ou aux protéines produites à l'étape (b) est réalisée par tout test fonctionnel de la ou desdites protéines déterminé de l'homme du métier. Si les acides nucléiques sont présents dans l'échantillon, la fonction est détectée ou dosée de manière directe ou indirecte par un ou plusieurs tests fonctionnels de la ou des protéines produites à l'étape (b). La détection et/ou la mesure de la fonction correspondant à la ou aux protéines produites à l'étapes (b) peut mettre en oeuvre à l'une des étapes (a) et/ou (b) et/ou (c) une ou plusieurs molécules rapporteur permettant de révéler la présence de la ou des fonctions analysées caractéristiques d'un processus ou d'un organisme.

La molécule rapporteur, si elle est présente, peut être tout substance capable de révéler directement ou indirectement la fonction connue recherchée codée par la ou les protéines exprimées à l'étape (b), comme une molécule d'acide nucléique, une protéine, un peptide, tel qu'un anticorps ou un mélange d'anticorps spécifique(s) d'une protéine et capable(s) de révéler son activité, ou un substrat ou une cascade de substrats, dont l'un est celui de l'enzyme correspondant à la fonction recherchée ou toute molécule chimique ou synthétique ou organique.

Ainsi, à l'étape (c), la mise en contact de la molécule rapporteur avec les protéines éventuellement exprimées à l'étape (b) peut être réalisée par addition de la molécule rapporteur dans le mélange réactionnel résultant de l'étape (b). Mais la molécule rapporteur peut aussi être présente dans le mélange réactionnel dès l'une des étapes (a) ou (b), soit sous sa forme finale de molécule rapporteur, soit, selon une forme particulière de réalisation de l'invention, sous la forme d'une molécule d'acide nucléique (ADN, ou ARN) correspondant au gène codant pour ladite molécule rapporteur, et alors désigné ci-après gène rapporteur. Dans ce mode de réalisation particulier, la molécule rapporteur sera produite lors de l'étape (b) conjointement avec la ou les protéines correspondant à la fonction recherchée.

Dans cette forme particulière de mise en oeuvre de la méthode de l'invention, le gène rapporteur est placé sous les mêmes séquences de contrôle de régulation de la transcription et de la traduction que le ou les gènes codant pour la ou les protéines correspondant à la fonction analysée de façon à être coexprimé avec ceux-ci et ainsi permettre à la molécule rapporteur d'être présente lors de l'étape (c). Le gène rapporteur avec ses séquences de régulation de la transcription et de la traduction est présent sous toute forme d'acide nucléique comme un vecteur d'expression *in vitro.*

A titre d'exemple, le gène rapporteur peut être le gène de la protéine GFP (Green Fluorescent Protein) ou celui de la beta-lactamase (TEM-1).

Dans le cas de la GFP, l'étape (c) comprend la détection et la quantification de l'émission de fluorescence. Ainsi, la méthode de l'invention permet par exemple de réaliser un test tel que la GFP n'est fluorescente que si la séquence cible est absente de l'échantillon d'acide nucléique étudié. Il convient de noter que le gène rapporteur de la GFP présente l'avantage de produire une protéine, dont l'activité est instantanément mesurable, ce qui permet un gain de temps supplémentaire.

Dans le cas de la beta-lactamase, l'étape (c) comprend la mesure de l'activité de cette enzyme, en incubant une fraction de la réaction de traduction dans un tampon contenant de la nitrocéphine. La nitrocéphine est une beta-lactamine chromogénique qui a la propriété de virer du jaune au rouge lorsqu'elle est hydrolysée par une beta-lactamase active. Un test rouge indique par exemple que la séquence cible est présente dans l'échantillon d'acide nucléique analysé.

Tout autre gène rapporteur peut être envisagé dans le cadre de la méthode de l'invention, comme par exemple ceux de la beta-galactosidase, la luciférase, la peroxydase ou une microperoxydase etc... . Il est à noter qu'un gène rapporteur correspondant à une protéine ayant une activité enzymatique présente une grande sensibilité dûe au coefficient enzymatique multiplicateur.

La méthode de l'invention offre donc un grand choix de mode de réalisation à partir de gènes et molécules rapporteurs choisis en fonction de la nature et de l'activité de la ou des protéines correspondant à la fonction recherchée.

La mesure de l'activité d'une ou plusieurs protéines à l'étape (c) peut être lue directement dans un lecteur de fluorimétrie si la mesure de la fonction met en oeuvre un fluorophore comme par exemple la GFP ou de colorimétrie si la mesure de la fonction met en oeuvre un chromophore comme par exemple la nitrocéphine. Mais on peut également envisager des mesures par absorbance, par viscosimétrie, par spectrophotométrie de masse ou tout autre méthode liée à la mesure de la fonction à l'étape c. Il est tout a fait envisageable de réaliser une lecture en continue de la fonction, si le test fonctionnel s'y prête.

Comme indiqué précédemment la fonction analysée peut correspondre à une seule protéine ou à plusieurs protéines, comme par exemple dans le cas d'un phénotype exprimé par un opéron. En conséquence, la méthode de l'invention accepte plusieurs formes de réalisation.

Lorsque la fonction détectée et/ou quantifiée correspond à une seule protéine, la méthode de l'invention comprend les étapes (a) à (c) suivantes :
a) la préparation, à partir de l'échantillon d'acide nucléique, de molécule d'acides nucléiques comprenant le gène codant pour la protéine correspondant au à ladite fonction en 5' dudit gène un promoteur d'ARN polymérase et un site de fixation des ribosomes, et éventuellement un terminateur d'ARN polymérase en 3' dudit gène,
b) la transcription et la traduction *in vitro* de la molécule d'acide nucléique préparée à l'étape (a), éventuellement en présence d'une ou plusieurs molécules rapporteurs permettant de révéler et/ou de mesurer l'activité, la présence ou l'absence de la protéine correspondant à ladite fonction,
c) la mise en contact des protéines éventuellement produites à l'étape (b) avec une ou plusieurs molécules rapporteurs, si celle(s)-ci n'étai(en)t pas présente(nt) à l'étape (b), permettant de révéler et/ou de mesurer l'activité, la présence de la protéine correspondant à ladite fonction.

Mais la méthode de l'invention peut aussi être appliquée à la détection et/ou à la quantification d'une fonction correspondant à un ensemble de protéines. Les gènes codant pour ces protéines peuvent être situés sur le même fragment d'ADN comme dans le cas d'un opéron, ou à différents endroits de l'ADN génomique comme dans le cas de certaines voies métaboliques.

Lorsque la fonction connue analysée correspond à plusieurs protéines, l'étape (a) de la méthode de l'invention admet les deux formes de mise en oeuvre suivantes :
i) Soit, lesdits gènes sont regroupés sous la forme d'un opéron, et alors l'étape (a) consiste à préparer à partir de l'échantillon d'acides nucléiques, une molécule d'acide nucléique comprenant les gènes (l'opéron) codant pour les protéines correspondant à ladite fonction, si ils sont présents dans les acides nucléiques de l'échantillon, en 5' de l'ensemble desdits gènes (de l'opéron) un promoteur d'ARN polymérase, éventuellement en 3' de l'ensemble desdits gènes (de l'opéron) un terminateur d'ARN polymérase, et pour chacun desdits gènes son site de fixation des ribosomes naturel.
ii) Soit, lesdits gènes sont séparés et alors l'étape (a) consiste à préparer, à partir de l'échantillon d'acide nucléique, une ou plusieurs molécules d'acide nucléique comprenant les gènes codant pour les protéines correspondant à ladite fonction, si ils sont présents dans les acides nucléiques de l'échantillon, en 5' de chacun desdits gène un promoteur d'ARN polymérase et un site de fixation des ribosomes, et éventuellement en 3' de chacun desdits gènes un terminateur d'ARN polymérase.

Dans la première forme de mise en oeuvre (i) ci-dessus, une mise en oeuvre préférée concerne le site de fixation des ribosomes de chacun des gènes, qui est son site de fixation des ribosomes naturel, et l'on préfère alors utiliser à l'étape (b) un extrait de traduction préparé à partir de l'organisme dont provient l'échantillon d'acides nucléiques ou d'un organisme phylogénétiquement proche.

Dans la seconde forme de mise en oeuvre (ii) ci-dessus, le site de fixation des ribosomes peut être le site naturel de chacun des gènes ou un autre site de fixation des ribosomes plus adapté à l'étape (b) de traduction.

Une variante de la première (i) et deuxième forme (ii) de mise en oeuvre ci-dessus, consiste à réaliser en parallèle ou simultanément, la méthode de l'invention décrite précédemment lorsque la fonction analysée correspond à une seule protéine, chaque étape (a) étant réalisée avec chacun des gènes. Une alternative à la réalisation en parallèle ou simultanée des méthodes de l'invention, consiste à réaliser séparément pour chacun des gènes les étapes (a) et (b), puis, pour la détection et/ou la mesure finale de la fonction impliquant chacune des protéines, à rassembler les produits des étapes (b) pour réaliser l'étape (c).

De même, lorsque la fonction analysée correspond à des protéines dont les gènes sont physiquement séparés sur le génome, la méthode de l'invention consiste à réaliser en parallèle ou simultanément, la méthode décrite précédemment lorsque la fonction analysée correspond à une seule protéine, chaque étape (a) étant réalisée avec chacun des gènes. Une alternative à la réalisation en parallèle ou simultanée des méthodes de l'invention, consiste à réaliser séparément pour chacun des gènes, les étapes (a) et (b), puis, pour la détection et/ou la mesure finale de la fonction impliquant chacune des protéines, à rassembler les produits des étapes (b) pour réaliser l'étape (c).

L'invention concerne aussi une méthode de quantification d'une fonction connue, à partir des acides nucléiques présents dans ledit échantillon, caractérisée en ce qu'elle comprend :
a) à c) la mesure de ladite fonction selon les étapes (a) à (c) ci-dessus, puis
d) la comparaison de la mesure de la fonction éventuellement présente dans l'échantillon effectuée à l'étape (c) avec une valeur étalon ou un ensemble de valeurs étalons de ladite fonction mesuré sur un ou plusieurs échantillons étalons selon un procédé de mesure identique ou équivalent à celui de l'étape (c).

Un échantillon étalon pour la réalisation de l'étape (d) ci-dessus peut être tout échantillon contenant :
- une quantité, avantageusement connue, du ou des gènes codant pour la ou les protéines correspondant à la fonction analysée par la méthode de l'invention, et qui sera alors soumis à un traitement de transcription traduction comme à l'étape (b), puis la fonction de la ou des protéines obtenue(s) sera mesurée selon un procédé de mesure identique ou équivalent à celui de l'étape (c),
- une quantité, avantageusement connue, de la ou des protéines correspondant à ladite fonction, laquelle sera mesurée selon un procédé de mesure identique ou équivalent à celui de l'étape (c),
- une quantité, avantageusement connue, d'un ou plusieurs organismes ou processus possédant le ou les gènes codant pour la ou les protéines correspondant à ladite fonction, laquelle sera mesurée selon un procédé de mesure identique ou équivalent à celui de l'étape (c).

L'échantillon étalon peut provenir d'un milieu identique ou différent de celui sur lequel les étapes (a) à (d) de la méthode de l'invention sont réalisées. Il peut s'agir du même milieu mais prélevé à un moment différent.

Dans une forme particulière de réalisation de la méthode de détection de l'invention, l'échantillon peut contenir lui-même l'étalon.

Elle peut être évaluée notamment par rapport à un seuil prédéterminé ou par rapport à une courbe standard permettant la comparaison des mesures de la fonction de l'étape (c) avec celles d'échantillons étalons.

Dans le cadre des différentes mise en oeuvre de la présente invention, la préparation de l'échantillon à l'étape (a) de la méthode de l'invention consiste à placer une ou plusieurs séquences d'acide nucléique codant pour la ou les protéines correspondant à la fonction analysée que l'on souhaite détecter ou quantifier sous le contrôle d'éléments nécessaires à la transcription et à la traduction *in vitro.*

Ainsi, à l'étape (a), les séquences de contrôle du ou des gènes codant pour la ou les protéines correspondant à la fonction détectée et/ou quantifiée selon le procédé de l'invention sont pour la transcription :
- un promoteur d'ARN en 5' du brin codant du ou desdits gènes,
- éventuellement un terminateur d'ARN polymérase en 3',
et pour la traduction :
- un site de fixation des ribosomes qui peut être ou non le(s) site(s) de fixation naturel(s) du ou des gènes.

Le promoteur (en 5') et le terminateur (en 3') s'il est présent, d'une ARN polymérase, sont par exemple ceux de l'ARN polymérase des phages T7, SP6, Qβ ou λ.

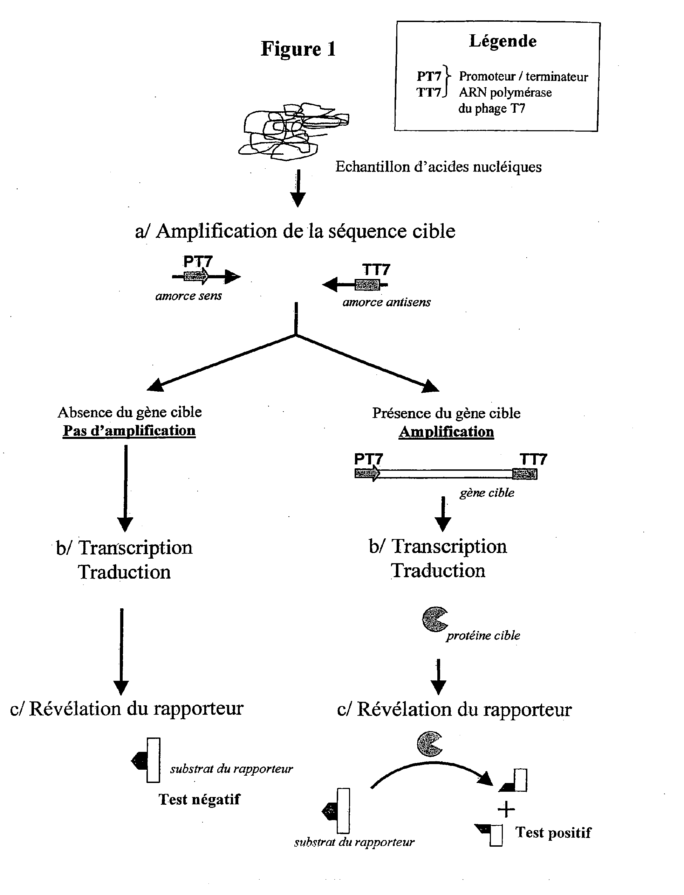

Une forme de mise en oeuvre avantageuse de l'étape (a) de la méthode de l'invention consiste à préparer la ou les molécules d'acide nucléique par une réaction d'amplification du ou des gènes codant pour la ou les protéines correspondant au phénotype analysé, à partir de l'échantillon d'acides nucléiques. Il peut s'agir d'une amplification par PCR ou par des techniques dérivés de la PCR du type RT-PCR, nested PCR, multiplex PCR, ou des techniques différentes de la PCR du type NASBA,SDA ou rolling circle et autres. Avantageusement cette préparation met en oeuvre un couple d'oligonucléotides ou un couple d'amorces spécifiques de la ou des molécules d'acide nucléique comprenant le ou les gènes codant pour la ou les protéines correspondant à la fonction analysée. Cette préparation par amplification est réalisée à l'aide d'une ou plusieurs paires d'amorces, chacune constituée pour l'exemple de la PCR (figure 1)et de la NASBA :
- pour l'amorce sens, de la séquence s'hybridant en amont d'une ou plusieurs molécules d'acide nucléique comprenant le ou les gènes codant pour la ou les protéines correspondant à la fonction analysée, et d'un promoteur d'ARN polymérase et éventuellement un site de fixation des ribosomes, et
- pour l'amorce antisens, de la séquence s'hybridant en aval d'une ou plusieurs molécules d'acide nucléique comprenant le ou les gènes codant pour la ou les protéines correspondant à la fonction analysée, et éventuellement d'un terminateur d'ARN polymérase.

L'étape (a) peut être réalisée par toute autre technique appropriée. En effet, la préparation de la molécule d'acide nucléique de l'étape (a) peut être réalisée par toute autre méthode connue de l'homme du Métier comme par exemple une coupure de restriction permettant de récupérer le ou les gènes d'intérêt suivie d'une ligation orientée avec les éléments de contrôle nécessaires à la transcription et à la traduction *in vitro* indiqués précédemment.

Lorsque l'invention concerne la détection ou la quantification d'au moins deux fonctions dans le même échantillon, quelque soit la technique de préparation de la ou des molécules d'acide nucléique l'étape (a), celle-ci peut être mise en oeuvre avec des amorces discriminantes ou non.

Ainsi, lorsqu'à l'étape (a) on utilise des amorces non discriminantes, alors à l'étape (c) on détecte et/ou l'on mesure lesdites fonctions par des tests fonctionnels permettant de différencier lesdites fonctions.

Ou, lorsqu'à l'étape (a) on utilise des amorces discriminantes, alors qu'à l'étape (c) on détecte et/ou l'on mesure lesdites fonctions par des tests fonctionnels permettant ou non de différentier lesdites fonctions.

Dans le cas de l'utilisation de couples d'amorces à l'étape (a) de la préparation de molécules d'acides nucléiques, différentes formes de mise en oeuvre de la méthode de l'invention peuvent être réalisées. Il est possible de combiner dans un seul tube ou non deux réactions de détection, et/ou quantification objet de la méthode de l'invention. L'étape (a) est réalisée alors avec les amorces nécessaires à l'amplification spécifique des gènes cibles. Dans ce cas, deux tests fonctionnels différents peuvent être utilisées pour détecter et/ou quantifier chacune des fonctions. Il est encore possible d'utiliser le même test fonctionnel pour les fonctions, et un résultat positif indique alors uniquement la présence de l'une ou de l'autre des protéines cibles. Il est donc possible d'utiliser autant de tests fonctionnels que de protéines cibles, chaque rapporteur différent permettant de révéler chacune des protéines cibles.

Il est possible d'utiliser des amorces discriminantes ou non à l'étape (a) et/ou des tests fonctionnels différents ou non à l'étape (c) de la méthode de l'invention (exemple I).

Des amorces discriminantes permettent d'isoler spécifiquement une séquence d'acide nucléique codant pour une protéine correspondant à une fonction représentative d'un processus ou d'un organisme, alors que les amorces non discriminantes ne permettent pas forcément d'isoler spécifiquement un gène codant pour une telle fonction.

Dans le cas de l'utilisation d'amorces non discriminantes à l'étape (a) de la méthode de l'invention, la spécificité du processus ou de l'organisme pourra être déterminée par un test fonctionnel ou par une combinaison de tests fonctionnels réalisés à l'étape (c) de la méthode de l'invention. Les amorces non discriminantes peuvent être universelles ou non, dégénérées ou non. On entend par amorces dégénérées, des amorces qui s'hybrident partiellement à la cible nucléotidique codant pour la protéine correspondant à la fonction recherchée. On entend également par amorces non discriminantes des pools de couples d'amorces discriminantes pour préparer à l'étape (a) la ou les molécules d'acide nucléique comprenant le ou les gènes codant pour la ou les protéines correspondant à la fonction recherchée présente dans un ou plusieurs processus ou un ou plusieurs organismes.

Il est possible de réaliser la méthode de l'invention en combinant des amorces discriminantes ou non à l'étape (a) avec des tests fonctionnels spécifiques ou non à l'étape (c) pour réaliser la détection et la quantification d'un processus ou d'un organisme. En effet, c'est l'interprétation de ces différents tests qui permet de déterminer la spécificité de la détection.

La méthode de l'invention permet d'analyser les différentes fonctions correspondant à l'expression de plusieurs mutants d'un même gène pouvant être contenus dans un même échantillon d'acides nucléiques de départ. A titre d'exemple de cette application de la méthode de l'invention, on peut citer les différents mutants du gène de la protéase du VIH contenus dans un échantillon d'un patient infecté par ce virus. La mise en oeuvre de la méthode de l'invention consiste alors à réaliser chaque étape (a) avec chacun des mutants dudit gène de façon à exprimer chacun de ceux-ci séparément. La séparation des mutants contenus dans l'échantillon peut être réalisée par clonage, dilution extrême ou par toute autre méthode connue de l'homme du Métier. Cette application de la méthode de l'invention consiste donc à analyser les différentes formes d'une fonction connue contenue dans un seul échantillon d'acides nucléiques. On entend par différentes formes d'un fonction connue, la manifestation de propriétés comparables, car tous les variants de la protéase du VIH ont une activité protéasique, mais distinguables les unes des autres, puisque chaque variant protéique de la protéase du VIH peut présenter une résistance spécifique à un antiprotéase.

L'invention se rapporte donc aussi à l'application de la méthode à la détection et/ou la quantification in vitro des différentes formes d'une fonction connue dans un échantillon d'acides nucléiques susceptibles de contenir le ou les gènes codant pour la ou les protéines correspondant à ces différentes fonctions. En effet, comme indiqué ci-dessus sur la protéase du VIH, l'échantillon d'un patient infecté par ce virus peut contenir plusieurs variants du virus chacun exprimant une protéase différente. Il est donc intéressant d'effectuer non seulement un diagnostic de la présence du virus par l'intermédiaire de la recherche de la fonction de la protéase du virus conformément à la méthode de l'invention, mais aussi de réaliser une analyse de la représentation des différents variants de cette protéase.

Sur la base de cet exemple sur le VIH, l'invention permet l'analyse *in vitro* des différentes formes d'une fonction connue correspondant à l'expression de plusieurs variants d'un gène pouvant être contenu dans un même échantillon d'acides nucléiques de départ. Ce but est atteint selon l'invention, en amplifiant éventuellement les différents variants, par exemple par culture cellulaire ou par amplification moléculaire, puis en isolant chaque gène, par exemple par clonage ou par dilution extrème, et en exprimant chacun de ces gènes conformément aux étapes (a) et (b) de la méthode de l'invention, et enfin en révélant de la ou des fonctions recherchée.

Les réactions de transcription et de traduction (étape b) peuvent être simultanées, ce qui signifie que la phase de traduction est réalisée simultanément avec la transcription, ou décomposée en deux étapes distinctes de transcription et de traduction.

Le découplage des étapes de transcription et de traduction permet d'optimiser les rendements de chaque étape, et ainsi de produire des quantités plus importantes de protéines, ce qui trouve toute son utilité dans le cas de la détection d'enzymes de faible activité spécifique.

Ce découplage permet aussi de normaliser la formation des produits à l'étape (b) et de pouvoir comparer ultérieurement les différentes fonctions exprimées.

Le découplage entre la transcription et la traduction permet également d'éviter les problèmes de dégradation de la matrice ADN par les nucléases si celle-ci a été préparée par PCR. En effet, les composants de la réaction de transcription sont moins contaminés par des nucléases, contrairement aux extraits de traduction.

Le découplage permet en outre l'emploi d'extraits de traduction différents selon l'origine de l'ADN criblé. En effet, la phase de traduction du transcrit est avantageusement réalisée avec un extrait de traduction de la même origine ou d'une origine proche de celle de l'échantillon biologique sur lequel est pratiqué le procédé de l'invention. Ainsi, on optimise l'adéquation entre l'origine des signaux de traduction des transcrits et l'extrait cellulaire pour une efficacité de traduction optimale. On peut citer à titre d'exemple l'utilisation d'un extrait de traduction préparé à partir de cellules eucaryotes pour la traduction d'une séquence cible eucaryote ou d'un extrait de traduction préparées à partir de mycobactéries non pathogènes pour la traduction de gènes de Mycobactéries pathogènes tel que l'intéine de RecA de Mycobacterium tuberculosis. Dans un autre cas de figure, l'extrait de traduction est préparé à partir d'organismes extrêmophiles pour la traduction d'un gène d'un même organisme ou d'un autre organisme extrêmophile du même type (thermophiles, halophiles, acidophiles, etc...). Ces extraits respectifs sont susceptibles d'améliorer l'efficacité du procédé. Ces extraits sont choisis pour leur capacité à traduire les transcrits.

Le procédé de l'invention est remarquable en ce qu'il met en oeuvre une adéquation entre la ponctuation d'expression des transcrits et les extraits de traduction utilisés. Ces extraits sont aussi caractérisés en ce que soit ils ne contiennent pas la propriété recherchée, soit ils la contiennent mais qu'elle n'est pas détectable dans les conditions de test réalisées pour détecter la fonction recherchée. Il s'agit par exemple de l'utilisation d'un extrait de traduction contenant une activité beta-galactosidase mésophile permettant de traduire un ARNm d'une beta-galactosidase thermophile et de la détection de l'activité de cette dernière à haute température, ce qui élimine l'activité beta-galactosidase mésophile.

En fonction de l'origine génétique des molécules d'acide nucléique préparées à l'étape (a), par exemple ADN de microorganismes Gram positifs, ou négatifs, d'eucaryotes, de virus etc..., et de la fonction testée, différents extraits de traduction peuvent donc être utilisés.

Une mise en oeuvre particulière du procédé de l'invention consiste à utiliser à l'étape (b) un extrait de traduction qui soit en fait un mélange de plusieurs extraits de traduction. Il peut s'agir par exemple d'extrait de traduction de E. coli surexprimant une protéine chaperon A mélangé avec un extrait de traduction de E. coli surexprimant une protéine chaperon B. Tout type de mélange est envisageable du moment qu'il correspond aux caractéristiques décrites ci-dessus. De la même manière, il est possible d'utiliser un extrait de traduction dans lequel est rajouté un ou plusieurs tRNAs spécifiques d'un ou de plusieurs codons. Les extraits de traduction ainsi obtenus permettent alors de traduire des mRNA comportant ces codons spécifiques, comme par exemple la traduction d'un mRNA contenant un codon ambre en rajoutant dans l'extrait de traduction un ou des tRNAs suppresseurs.

Le traitement de l'étape (b) avec un extrait de traduction peut aussi être réalisé avec un extrait standard de traduction quelque soit l'origine de l'échantillon comme par exemple un extrait d'E. coli et/ou tout(s) autre(s) extrait(s) cellulaire(s) supplémentés ou non par des molécules intéressantes comme celles, par exemple, indiquées précédemment (tRNA, chaperon...) .

Il est également possible d'ajouter à l'extrait de traduction de l'étape (b) une ou plusieurs substances favorisant un repliement ou une maturation plus efficace des protéines exprimées, comme par exemple des chaperons, des détergents, des sulfobétaïnes, des extraits membranaires, etc... .

Dans le cas où la séquence cible est de type eucaryote, la réaction de transcription de l'étape (b) peut être complétée par une réaction de splicing et de maturation in vitro des ARNm en ajoutant un extrait nucléaire au mélange réactionnel.

Dans le cas particulier où le gène rapporteur est coexprimé avec la ou les protéines codant pour la ou les fonctions, la réaction de transcription et de traduction de l'étape (b) est standardisée de façon à éviter des résultats correspondant à des faux positifs ou à des faux négatifs :
- d'une part, pour que la protéine cible codant pour la fonction ait le temps d'agir au niveau de la mesure de la fonction à l'étape (c ) comme par exemple d'agir sur le rapporteur pour l'inhiber par exemple, ou pour éviter de se placer dans des conditions où le rapporteur serait en plus grande quantité que la protéine cible.
- d'autre part, pour se placer dans des conditions d'activité à la fois pour la protéine cible (correspondant à la fonction) et le rapporteur si il est nécessaire au test de mesure à l'étape (c)."

La méthode de détection et/ou de quantification d'une fonction connue objet de l'invention est remarquable en ce qu'elle permet d'éviter les faux positifs dus à des interférences des composés présents dans l'échantillon de départ lors de la détection et/ou la mesure de la fonction analysée à l'étape (c). En effet, du fait du principe même de la méthode de l'invention, les interactions potentielles des composés du milieu initial sur la détection et/ou la quantification de la fonction à détecter et/ou quantifier sont réduites (par exemple par dilution dudit milieu initial au fur et à mesure des différentes étapes du procédé de l'invention, mais surtout par la spécificité du test fonctionnel utilisé à l'étape (c) du procédé de l'invention). En outre, la méthode de l'invention s'attachant à détecter et/ou quantifier une fonction, toutes les molécules d'acide nucléique codant pour des protéines non fonctionnelles ou toutes les molécules d'acide nucléique présentant des homologies de séquence avec la séquence recherchée mais codant pour une fonction différente ne seront pas détectés, ce qui est un des avantages majeurs de la méthode de l'invention par rapport aux techniques de l'art antérieur.

La méthode de l'invention offre l'avantage de pouvoir détecter spécifiquement une ou plusieurs séquences cibles dans un échantillon à analyser et de travailler ultérieurement directement sur cette ou ces séquences cibles. La sensibilité de cette méthode s'explique par le coefficient multiplicateur des étapes (b) et (c) correspondant respectivement à la transcription, à la traduction du ou des gènes préparés à l'étape (a) puis à la détection et/ou la mesure de la fonction correspondant à la ou aux protéines produites à l'étape (b). De plus, pour augmenter la sensibilité, la méthode de l'invention peut passer après l'étape de transcription par une étape d'amplification des transcrits par toute technique connue de l'homme de métier comme la NASBA (nucleic Acid Sequence-based Amplification), la TMA (Transcription Mediated Amplification) avant l'étape de traduction. La méthode de l'invention est en outre rapide et reproductible, car toutes les réactions sont réalisées *in vitro,* ce qui permet de standardiser la détection et de s'affranchir de tous les problèmes liés aux tests de diagnostic *in vivo*, comme la diffusion membranaire, la toxicité cellulaire ou l'état physiologique des cellules.

Il convient de remarquer que dans cette application de la méthode de l'invention, la fonction recherchée est mise en évidence grâce à l'activité de la protéine cible qui est exprimée *in vitro*. Dans ce cas, la méthode de l'invention permet non seulement de mettre en évidence la présence d'une ou plusieurs fonctions d'un processus ou d'un organisme, mais aussi de caractériser l'activité de la ou des protéines correspondantes. Cette caractérisation, peut être aussi désignée phénotypage de la protéine ou des protéines exprimées selon le procédé de l'invention, par opposition à la génomique qui est la caractérisation de la composition en acides nucléiques de la séquence cible. Le diagnostic de séquences cibles correspond alors également au diagnostic de la fonction et à sa caractérisation. On entend par exemple par caractérisation, la définition du spectre d'inhibition d'une protéine cible par des inhibiteurs spécifiques ou la définition d'une gamme de pH dans laquelle la protéine cible est active. Si la fonction est une activité enzymatique, il peut s'agir de l'analyse des conditions optimales de fonctionnement (pH, température, concentration en sels), des paramètres cinétiques (Vm, Km), des paramètres d'inhibition (Ki). Si la fonction est une affinité, il peut s'agir de la détermination du Kd, ou de la molécule ayant le plus d'affinité pour cette protéine. Il peut s'agir encore de la détermination de la taille de la protéine traduite ou de l'ARNm transcrit, et éventuellement du séquençage du gène correspondant.

A titre d'exemple, cette méthode présente un intérêt tout particulier dans le diagnostic de la protéine cible correspondant à la protéinase à acide aspartique du virus HIV pour étudier consécutivement sa sensibilité à différents inhibiteurs spécifiques de protéinases ou antiviraux, lorsque la séquence cible correspondante est présente dans un échantillon d'acide nucléique.

La caractérisation d'une fonction représente la détermination des caractéristiques de la dite fonction et en particulier l'identification de substance(s) capable(s) de modifier ladite ou lesdites fonction(s). On entend par substance des polynucléotides, des peptides, des protéines, les ions, des molécules ou des compositions chimiques naturelles ou synthétiques, des hormones, des composés aromatiques, des anticorps, des fragments d'anticorps, des gènes, des récepteurs cellulaires, des acides aminés, des glycopeptides, des lipides, des glycolipides, des sucres, des polysaccharides, etc... . , capables de modifier l'activité d'une ou plusieurs fonctions d'un organisme ou d'un processus. Ces dites substances pourront correspondre à une ou plusieurs têtes de séries. Il peut s'agir de tout agent capable de modifier la ou les fonctions comme des antiviraux, des inhibiteurs, des stimulants, des conditions physico-chimiques, des radiations ou des traitements thermiques. Cette caractérisation peut se faire en série avec la détection et/ou la quantification ou de manière séquentielle. Cette caractérisation de fonction permet d'effectuer des analyses statistiques. En effet, après avoir identifié différentes fonctions ou différents variants fonctionnels, il est possible d'étudier individuellement par exemple leur résistance à une ou plusieurs substances. Cette forme de mise en oeuvre de la méthode de l'invention est avantageusement réalisée sur une plaque de microtitration ou sur une puce pour étudier par exemple le comportement de différents variants génétiques de la fonction protéinase à acide aspartique de HIV1 vis-à-vis de plusieurs antiviraux.

En conséquence, la méthode de l'invention peut comprendre après l'étape (c), un test de ladite fonction avec une substance.

La détection et/ou la quantification de différentes fonctions présentes dans un échantillon selon la méthode de l'invention présentent un avantage certain dans le suivi d'un organisme ou d'un processus. En effet, la méthode de l'invention permet de détecter et/ou de quantifier par exemple les fonctions d'un virus et d'un composant cellulaire. Cette mise en oeuvre de l'invention trouve par exemple un intérêt tout particulier dans la détection et/ou la quantification de la présence d'un pathogène dans un organisme (virus ou bactérie), qui peut( peuvent) être corrélé(es) à la détection et/ou la quantification d'une fonction spécifique dudit organisme infecté ou de la réponse à l'infection de l'organisme infecté (par exemple production de l'alpha-1 acid glycoprotein (AAG) dans le cas de l'infection par le virus du HIV).

La méthode de l'invention permet en outre d'effectuer des suivis d'une ou plusieurs fonctions dans différents échantillons. A titre d'exemple, on peut citer le suivi des différents variants fonctionnels de différentes fonctions caractéristiques d'une infection par le virus HIV. L'apparition des différents variants fonctionnels est caractéristique des phénomènes de résistances à un ou plusieurs antiviraux. Selon le développement de cette résistance, différents tissus porteront différents variants génétiques du virus. Le suivi des différents variants génétiques dans différents tissus donne alors une indication de la propagation de l'infection. Selon les résultats de détection des différents variants génétiques dans différents tissus, le médecin pourra juger de l'état immunitaire d'un patient et de sa capacité à lutter contre des maladies opportunistes et envisager pour son malade outre un traitement par des antiviraux, un traitement par des antibiotiques et ou des antifongiques.

Compte tenu de la facilité, du faible coût et de la rapidité de la méthode de l'invention, un suivi de la ou des fonctions peut être mis en oeuvre de manière à effectuer périodiquement la détection et la quantification de la ou desdites fonctions. La comparaison des résultats obtenus à différents temps et leur interprétation permet un suivi dans le temps de l'évolution d'un processus.

Ces différents suivis permettent au praticien de comprendre le développement d'un processus ou d'un organisme. L'interprétation des résultats permettra au praticien qui met en oeuvre le procédé de l'invention de prendre une décision.

Les étapes du procédé de l'invention peuvent être réalisées successivement ou non sans interruption ou non par le même opérateur. Il peut être réalisé sur un dispositif automatisé intégrant chacune des étapes, ou peuvent être réalisées de façon discontinues, éventuellement par des opérateurs différents. Les échantillons d'acides nucléiques peuvent être alors placés sur un support pouvant correspondre par exemple à une biopuce ou une plaque de microtitration contenant plusieurs dizaines à plusieurs milliers d'emplacements. Ces supports sont conçus pour permettre :
- la préparation des séquences cibles (étape a),
- l'initiation des réactions de transcription et de traduction (étape b) et pour la révélation de la molécule rapporteur (étape c).

En conséquence, l'invention concerne un dispositif comprenant un agencement d'un ou plusieurs supports, de robots et d'un lecteur desdits supports pour la réalisation des étapes de la méthode décrite précédemment.

L'invention concerne aussi un kit pour la mise en oeuvre des méthodes décrites précédemment.

Dans une première forme de réalisation un kit selon l'invention comprend : les moyens de révélation de la fonction, une ARN polymérase, des séquences nucléotidiques pour la préparation des molécules d'acides nucléiques comprenant le ou les gènes permettant l'expression de la ou des protéines correspondant à la fonction détectée et/ou quantifiée, les quatre nucléotides triphosphates, les mélanges nécessaires à ladite préparation, à la transcription et à la traduction, éventuellement des témoins.

Dans une seconde forme de réalisation un kit selon l'invention comprend :
- éventuellement les produits nécessaires à la préparation des molécules d'acides nucléiques comprenant le ou les gènes permettant l'expression de la ou des protéines correspondant à la fonction détectée et/ou quantifiée,
- tout support comme plaque de microtitration ou puce contenant : les moyens de révélation de la fonction, une ARN polymérase, les quatre nucléotides triphosphates, les mélanges de transcription et de traduction, éventuellement des témoins.

Les kits et les supports peuvent être envisagés pour la détection et/ou la quantification simultanément ou non d'une ou plusieurs fonctions correspondant à un ou plusieurs processus ou un ou plusieurs organismes.

L'invention a donc aussi pour objet un support présentant une série d'emplacements pour la mise en oeuvre d'une méthode de l'invention, caractérisé en ce que chacun desdits emplacements permet la détection et/ou la mesure d'une fonction différente.

D'autres avantages et caractéristiques de l'invention apparaîtront dans les exemples de réalisation de l'invention, qui suivent et qui se réfèrent aux dessins en annexe dans lesquels :
- La figure 1 est une représentation schématique de la méthode de l'invention.
- La figure 2 est une représentation schématique de l'application de la méthode de l'invention au diagnostic du virus HIV.
- La figure 3 est une représentation schématique de l'application de l'invention au diagnostic de Mycobacterium tuberculosis.
- La figure 4 représente la détection de l'activité totale ou de l'activité des germes polyrésistants par la méthode de l'invention.
- La figure 5 représente la détection de la charge virale par la méthode de l'invention.

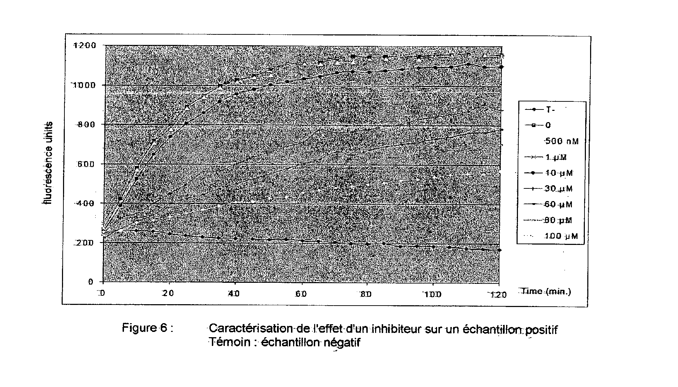

- La figure 6 représente la caractérisation de l'effet d'un inhibiteur sur un échantillon positif par rapport à un témoin négatif par la méthode de l'invention.

### Exemple I : Exemples de mises en oeuvre de la méthode de l'invention avec différents couples d'amorces à l'étape (a) de préparation et/ou différents tests fonctionnels à l'étape (c) de détection et de quantification d'une ou plusieurs fonctions.

Les cas ci-après permettent d'illustrer différentes possibilités de la méthode de l'invention.

### 1) Cas No. 1 : Les amorces utilisées à l'étape (a) du procédé de l'invention permettent de détecter et de quantifier l'activité réverse transcriptase du virus HIV.

Le tableau 1 ci-dessous montre que l'utilisation d'amorces discriminantes à l'étape (a) de la méthode de l'invention permet de détecter par un test fonctionnel à l'étape (c) :
- L'absence de la fonction réverse transcriptase dans l'échantillon 3, donc l'absence du virus HIV.
- La présence de la fonction réverse transcriptase dans les échantillons 1 et 2, donc la présence du virus HIV dans les échantillons 1 et 2. Le virus HIV est présent en plus grande quantité dans l'échantillon 2 que l'échantillon 1.

**Tableau 1**

| | Echantillon 1 | Echantillon 2 | Echantillon 3 |
|---|---|---|---|
| Amorces discriminantes utilisées à l'étape (a) | + | +++ | - |

### 2) Cas n° 2 : Des amorces non discriminantes sont utilisées à l'étape (a) de la méthode de l'invention pour détecter dans chaque échantillon la présence potentielle d'une activité réverse transcriptase.

Le tableau 2 ci-dessous montre que l'utilisation d'amorces non discriminantes à l'étape (a) de la méthode de l'invention permet de détecter par un test fonctionnel à l'étape (c) :
- L'absence de fonction réverse transcriptase dans l'échantillon 3, donc l'absence de virus.
- La présence de la fonction réverse transcriptase dans les échantillons 1 et 2, donc la présence de virus possédant une fonction réverse transcriptase dans les échantillons 1 et 2. La fonction réverse transcriptase est présente en plus grande quantité dans l'échantillon 2 que l'échantillon 1.

Le tableau 2 ci-dessous montre aussi que l'utilisation d'amorces discriminantes à l'étape (a) de la méthode de l'invention permet de détecter par un test fonctionnel à l'étape c) :
- La présence d'HIV dans l'échantillon 1.
- La présence d'HBV dans l'échantillon 2.

**Tableau 2**

| | Echantillon 1 | Echantillon 2 | Echantillon 3 |
|---|---|---|---|
| Amorces non discriminantes | + | +++ | - |
| Amorces spécifiques de la RT d'HIV | + | - | - |
| Amorces spécifiques de la RT d'HBV | - | +++ | - |

### 3) Cas n° 3 : Des tests fonctionnels différents sont mis en oeuvre à l'étape c du procédé de l'invention pour détecter une polymérase en fonction de sa thermorésistance.

Le tableau 3 ci-dessous montre que la méthode de l'invention permet de détecter par différents tests fonctionnels à l'étape (c) :
- La présence d'une polymérase préférentielle active à 100°C dans l'échantillon 1.
- La présence d'une polymérase active à 60°C dans l'échantillon 2.
- La présence d'une polymérase uniquement active à 100°C dans l'échantillon 3.

**Tableau 3**

| | Echantillon 1 | Echantillon 2 | Echantillon 3 |
|---|---|---|---|
| Test fonctionnel à 60 ° | + | +++ | - |
| Test fonctionnel à 100 ° | ++++ | + | +++ |

### 4) Cas n° 4 : Un test fonctionnel peut être réalisé en présence de différents substrats pour détecter et quantifier les différentes fonctions présentes dans un échantillon. Ce test fonctionnel peut permettre par exemple de détecter une activité polymérase.

Le tableau 4 ci-dessous montre que la méthode de l'invention permet de détecter par un test fonctionnel réalisé à l'étape (c) :
- La présence d'une activité ADN polymérase ADN dépendante et ARN dépendante dans l'échantillon 1.
- La présence d'une activité ADN polymérase ADN dépendante dans l'échantillon 2.
- La présence d'une activité ADN polymérase ARN dépendante dans l'échantillon 3.
- Le test fonctionnel en l'absence de substrat est un témoin négatif permettant de valider la détection.

**Tableau 4**

| | Echantillon 1 | Echantillon 2 | Echantillon 3 |
|---|---|---|---|
| Test fonctionnel + ADN | + | + | - |
| Test fonctionnel + ARN | + | - | + |
| Test fonctionnel sans substrat | - | - | - |

### 5) Cas n° 5 : Ce cas représente par exemple la détection du virus HIV dans des échantillons 1, 2 et 3 sanguins. Deux tests fonctionnels sont réalisés pour contrôler la spécificité de notre détection.

Le tableau 5 ci-dessous montre que L'utilisation de pool d'amorces discriminantes à l'étape a) de la méthode de l'invention sur différents échantillons sanguins permet de détecter par des tests fonctionnels différents réalisés à l'étape (c) :
- La présence d'HIV dans l'échantillon 1
- L'absence d'HIVdans l'échantillon 3
- Une erreur dans l'échantillon 2

**Tableau 5**

| | Test fonctionnel permettant de révéler l'activité réverse transcriptase | Test fonctionnel permettant de révéler l'activité protéinase à acide aspartique |
|---|---|---|
| Test 1 | + | + |
| Test 2 | + | - |
| Test 3 | - | - |

### 6) Cas n° 6 : Ce cas concerne la détection et la quantification des microorganismes suivants

- Aeromonas hydrophila se caractérise par les activités ONPG, LDC, ESC, OX.
- Escherichia coli 1 se caractérise par ONPG, LDC, ODC, UE.
- Salmonella arizonae se caractérise par ONPG, LDC, ODC.
- Viblrio alginolyticus se caractérise par LDC, ESC, OX.

Le tableau 6 ci-dessous montre que l'utilisation de pool d'amorces discriminantes à l'étape (a) de la méthode de l'invention sur différents échantillons permet de détecter par des tests fonctionnels différents a l'étape (c) et en fonction de différentes cartes d'identité fonctionnelles de microrganisme :
- La présence d'aeromonas hydrophila dans l'échantillon a.
- La présence d'E. coli 1 dans l'échantillon b.
- L'absence de microrganisme dans l'échantillon c
- La présence de Vibrio alginolyticus dans l'échantillon d
- La présence de Salmonella arizonae dans l'échantillone.

**Tableau 6**

| | ONPG | LDC | ODC | UE | ESC | OX |
|---|---|---|---|---|---|---|
| Echantillon a | +++ | + | - | - | + | +++ |
| Echantillon b | +++ | +++ | ++ | + | - | - |
| Echantillon c | - | - | - | - | - | - |
| Echantillon d | - | + | - | - | ++ | +++ |
| Echantillon e | +++ | +++ | +++ | - | - | - |

On entend dans le tableau 6 par :
- ONPG : beta D- galactosidase
- LDC : Lysine décarboxylase
- ODC : Ornitine décarboxylase
- URE : uréase
- ESC : beta-galactosidase
- OX : cytochrome oxydase

### Exemple II : Diagnostic du virus HIV.

Parmi les protéines virales nécessaires au cycle de multiplication du virus du HIV, on peut noter la protéinase à acide aspartique. La présence de cette protéine peut servir à diagnostiquer l'infection d'un patient par le rétrovirus.

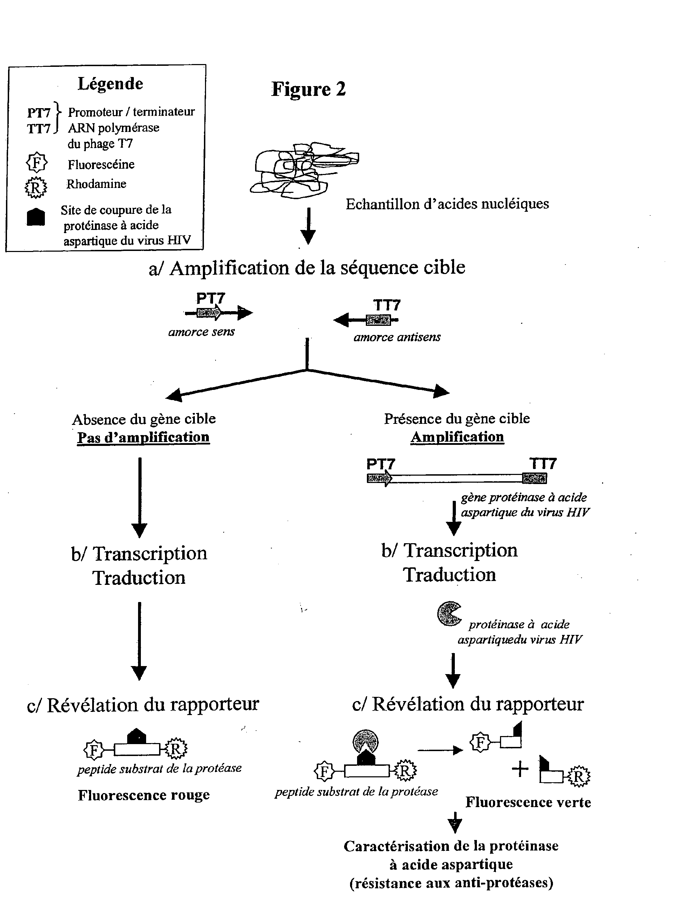

Comme montré à la figure 2, après une extraction des acides nucléiques d'un prélèvement sanguin, une étape d'amplification permet d'amplifier le gène de la protéinase à acide aspartique de HIV si le patient est infecté par le virus et d'intégrer le promoteur, le terminateur de la T7 ARN polymérase et un site RBS à la séquence cible.

La molécule rapporteur préparée à l'étape (a) est un peptide contenant des sites de coupure spécifiques de la protéinase de HIV. Ce peptide est lié covalemment respectivement en N et C terminal à une molécule fluorescente (comme par exemple la fluoresceïne) et à un quencher de cette dernière (comme par exemple la rhodamine).

Le gène cible amplifié est placé dans un tube chargé avec un mélange de transcription/traduction couplé (composants nécessaires à la transcription et à la traduction). Puis on ajoute la molécule rapporteur. Avantageusement, le milieu réactionnel de transcription et de traduction est composé soit d'un extrait cellulaire de traduction de E. coli, soit d'un extrait cellulaire de traduction de cellules eucaryotes.

Si la protéinase à acide aspartique est absente de l'échantillon testé, le rapporteur n'est pas clivé. La fluoresceïne et la rhodamine se trouvent alors dans un environnement suffisamment proche pour assurer un transfert d'énergie de type FRET (fluorescence resonance energy of transfert). Après exposition du peptide rapporteur à une longueur d'onde donnée (environ 490 nm), seule la fluorescence rouge du quencher sera détectable.

Si la protéinase à acide aspartique est présente dans l'échantillon testé, le rapporteur est clivé. La fluoresceïne et la rhodamine sont alors trop éloignés pour assurer un transfert d'énergie. Après exposition des produits de clivage à une longueur d'onde donnée (environ 490 nm), seule la fluorescence verte de la fluoresceïne est détectable.

Des témoins négatif et positif sont réalisés en parallèle. Il correspondent respectivement à une fraction d'une réaction PCR réalisée sur de l'ADN d'un patient sain et d'un patient infecté.

Après avoir diagnostiqué la présence du virus chez un patient, cette méthode permet un test consécutif de caractérisation de la protéinase à acide aspartique : selon la souche responsable de l'infection virale, la protéinase à acide aspartique présente une sensibilité variable à différents inhibiteurs spécifiques. Un tel phénotypage de la protéinase à acide aspartique permettra d'adapter la thérapie du patient directement après le diagnostic de l'infection virale. Pour cela, il suffit d'incuber une fraction de la réaction d'amplification dans différentes réactions de transcription/traduction, chacune contenant un inhibiteur spécifique de protéinase à acide aspartique utilisé actuellement dans des thérapies antivirales, et d'effectuer ensuite le test d'activité de la protéinase à acide aspartique avec le peptide FRET.

### Exemple III : Diagnostic de Mycobacterium tuberculosis.

Les méthodes classiques de diagnostic de la tuberculose et des infections mycobactériennes reposent sur l'examen microscopique et sur la culture des échantillons. Pour palier à la lenteur du diagnostic classique, de nouvelles techniques ont été développées.

Dans le cadre de la méthode de l'invention, le diagnostic du pathogène est réalisé en utilisant par exemple comme cible une de ses intéines. Les intéines sont des introns protéiques récemment découverts à l'intérieur de séquences protéiques. Les intéines possèdent toute l'information nécessaire à leur propre excision. Ce processus récemment mis en évidence est comparable à l'épissage des ARN prémessagers. Ainsi, par convention, la séquence protéique interne est appelée intéine et les deux séquences externes, extéines (Perler, F.B., Davis E.O., Dean, G.E., Gimble, F.S., Jack, W.E., Neff, N., Noren, C.J., Thorner, J. et Belfort, M. (1994). Protein splicing elements : inteins and exteins - a definition of terms and recommended nomenclature. Nucl. Acids Research 22, 1125-1127). Ce phénomène d'excision protéique spécifique est très rapide. Ces intéines ont été découvertes dans des groupes de gènes très différents et elles sont à la fois présentes chez les procaryotes et les eucaryotes. La protéine RecA de Mycobacterium tuberculosis contient un tel élément. D'autres Mycobactéries pathogènes peuvent posséder de telle séquence dans RecA comme Mycobacterium Leprae. Néanmoins, ces deux introns protéiques sont différents en taille, en séquence et dans leur localisation, ce qui les rend très spécifiques (Davis, E.O., Thangaraj, H.S., Brooks, P.C. et Colston, M.J. (1994). Evidence of selection for protein introns in the recAs of pathogenic mycobacteria. EMBO J. 13 (4), 699-703). En outre, ces intéines peuvent posséder une activité endonucléase, capable de reconnaître spécifiquement un mégasite.

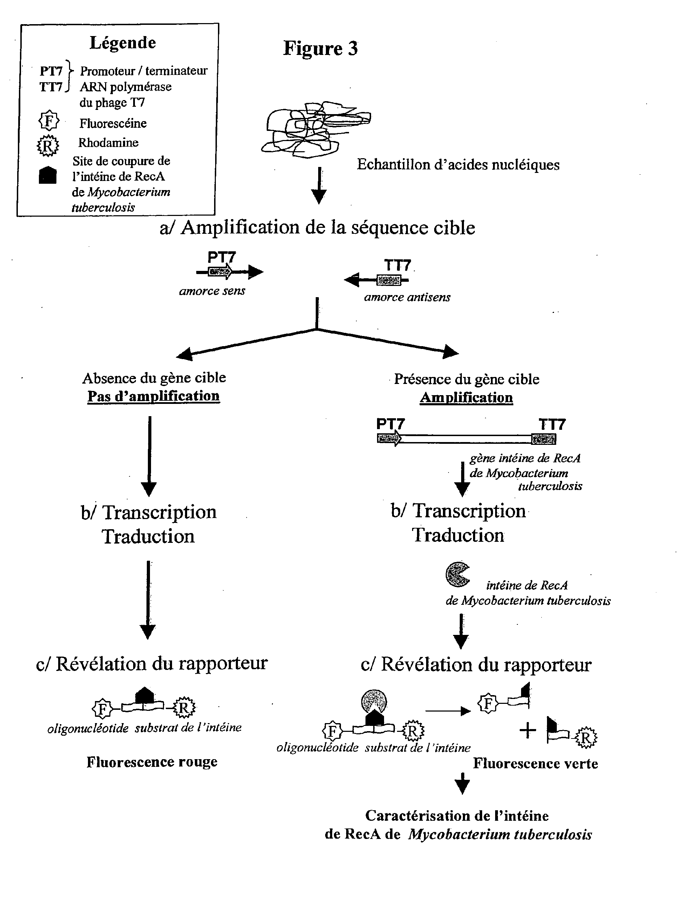

Le test montré à la figure 3 est le suivant. Après extraction de l'ADN de l'échantillon à analyser, une PCR permet d'amplifier le gène cible codant pour l'intéine en lui intégrant le promoteur, le terminateur de la T7 ARN polymérase et un site RBS. Le produit de PCR est ensuite incubé dans un mélange de transcription/traduction. Le rapporteur est ensuite ajouté. Ce rapporteur correspond à une séquence polynucléotidique caractérisé par la présence en 5' et en 3' respectivement d'une molécule de fluoresceine et de Rhodamine liées de façon covalente à l'ADN et en son centre du site de coupure spécifique de l'intéine de RecA de Mycobacterium tuberculosis.

Lorsque l'échantillon est contaminé par Mycobacterium tuberculosis, l'intéine produite dans la réaction de transcription/traduction, clive le rapporteur et empêche un transfert d'énergie de type FRET (décrit dans l'exemple précédent). Après exposition de la réaction de transcription/traduction à environ 490 nm, une coloration verte de l'échantillon est observée. En absence de contamination, le rapporteur n'est pas clivé et après exposition de la réaction de transcription/traduction à environ 490 nm, une coloration rouge de l'échantillon est observée.

Un témoin négatif est constitué d'une fraction de réaction PCR réalisée sur de l'ADN ne contenant aucun pathogène. Un témoin positif est constitué d'un fraction de réaction PCR réalisées sur de l'ADN d'un échantillon contaminé par le pathogène recherché.

### Exemple IV : Détection spécifique d'un élément au sein d'un échantillon contenant des éléments similaires.

### 1) Détection spécifique d'un élément au sein d'un échantillon contenant des éléments similaires.

Les β-lactamines (pénicillines et céphalosporines) représentent la classe d'antibiotiques la plus utilisée en thérapie anti-infectieuse. Depuis l'introduction en thérapie de ces molécules, de nouvelles résistances à ces composés se développent, favorisant l'expansion des infections nosocomiales. Parmi les différents mécanismes de résistance acquis par les germes, un des plus importants consiste à produire une enzyme (β-lactamase TEM-1 (Sutcliffe J.G., 1978, Nucleotide sequence of the ampicillin resistance gene of Escherichia coli plasmid pBR322, Proc. Natl. Acad. Sci. USA, 75, 3737-3741) pour certaines entérobactéries par exemple, ou beta-lactamase PSE pour Pseudomonas aeruginosa) capable d'hydrolyser l'antibiotique avant qu'il n'ait pu agir. Il existe quelques inhibiteurs de β-lactamases utilisés en synergie avec un antibiotique, mais au fur et à mesure de leur utilisation de nouvelles formes de β-lactamases résistantes à ces inhibiteurs sont apparues.

La détection de la fonction de ces enzymes représente donc un excellent test de diagnostic de la présence de germes résistants aux antibiotiques. D'autre part, la détection spécifique de beta-lactamases résistantes aux inhibiteurs est particulièrement avantageuse, car elle permet de savoir directement si un échantillon est contaminé par des germes polyrésistants (antibiotiques et inhibiteurs), ce qui permet d'adapter rapidement la thérapie.

Cette expérience permet la détection spécifique de germes polyrésistants (résistance aux antibiotiques et résistance à un inhibiteur de beta-lactamase : l'acide clavulanique) exprimant un variant résistant de TEM-1, dans un échantillon contaminé par des germes monorésistants (résistance uniquement aux antibiotiques).

10 µl de culture à D0600=2.3 ont été centrifugés et chaque culot a été repris dans 12 µl de tampon de lyse cellulaire (10 mM Tris HCl pH 7.5, 1 mM EDTA, 50 µg/ml protéinase K) et incubé 15 minutes à 55°C puis 15 minutes à 80°C. 9 µl de chacun de ces lysats ont été ajoutés à un mélange d'amplification PCR permettant d'amplifier le gène conférant la monorésistance et le gène conférant la polyrésistance ainsi que les séquences permettant leur expression *in vitro.* 5 µl de chaque amplification PCR ont été utilisés pour réaliser une transcription in vitro comme décrit par Gurevich et al. (Gurevich V.A., Pokrovskaya I.D., Obukhova T.A. et Zozulya S., 1991, Preparative in vitro mRNA synthesis using SP6 and T7 polymerases, Anal. biochem., 195, 207-213) 10 µl de chaque transcription ont été utilisés pour traduire *in vitro* les protéines comme décrit par Zubay (Zubay G., 1973, *In vitro* synthesis of protein in microbial systems, Ann. Rev. Genet., 7, 267-287) sur un volume final de 100 µl.

**Tableau 8**

| Echantillon | 1 | 2 | 3 | 4 | 5 | 6 | 7 |
|---|---|---|---|---|---|---|---|
| % de germes résistants aux antibiotiques | 100 | 90 | 70 | 50 | 30 | 10 | 0 |
| % de germes résistants aux antibiotiques et à l'acide clavulanique | 0 | 10 | 30 | 50 | 70 | 90 | 100 |

Deux types de tests d'activité ont été effectués :
- une mesure de l'activité "totale" bêtalactamase en utilisant comme substrat un antibiotique chromogénique : la nitrocéphine. 10 µl du mélange de traduction ont été incubés à 37°C dans un volume final de 1 ml de tampon de révélation de l'activité (concentration finale : NaP 50mM pH 7.0, 100µg/ml de nitrocéphine et 0.25 mM DMSO). La réaction a été suivie par spectrophotométrie à 486 nm.
- une mesure de l'activité "résistance à l'acide clavulanique" en utilisant comme substrat un antibiotique chromogénique : la nitrocéphine plus une concentration donnée d'acide clavulanique. 10 µl du mélange de traduction ont été incubés à 25°C pendant 3 minutes dans un volume final de 1 ml de tampon I (concentration finale : NaP 50mM pH 7.0, 1 µM d'acide clavulanique). Puis 100µg/ml de nitrocéphine et 0.25 mM final de DMSO ont été rajoutés, et la réaction a été suivie par spectrophotométrie à 486 nm.

Les résultats sont exposés dans le tableau 8 qui indique la composition des échantillons.

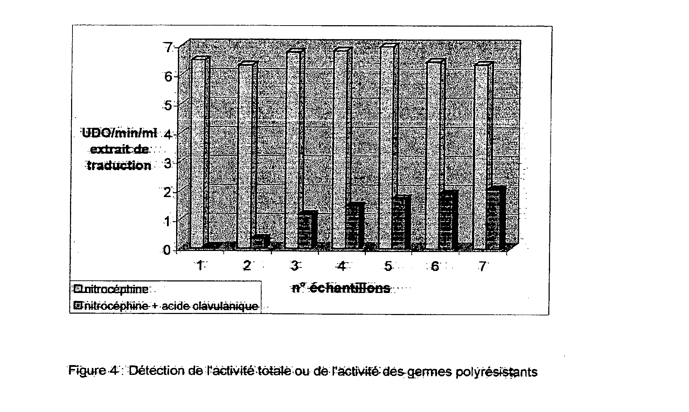

La figure 4 indique que l'activité totale beta-lactamase est détectée dans tous les échantillons avec un niveau comparable d'activité : le test permet donc de détecter simultanément deux fonctions (résistante aux antibiotiques et polyrésistance antibiotiques/acide clavulanique).

Lorsque le test de détection est réalisé en présence d'acide clavulanique, aucune activité n'est détectée dans l'échantillon 1 (0% de germes ayant la double résistance), et une activité croissante est détectée pour les échantillons 2 à 7 contenant de 10 à 100 % de germes ayant la double résistance. Ce test de détection fonctionnel est donc hautement discriminant puisqu'il permet de détecter l'activité des germes polyrésistants au sein d'une population de germes monorésistants.

Il est à noter que la différence entre l'activité totale détectée dans un échantillon et celle détectée uniquement pour les germes polyrésistants n'est pas égale à l'activité des germes monorésistants, car les enzymes de ces deux types de germes n'ont pas la même activité spécifique. En connaissant ces activités spécifiques, il serait possible de faire cette corrélation et de déduire l'activité des germes monorésistants en connaissant juste l'activité totale et celle des germes polyrésistants.

La méthode de l'invention permet donc :
- de réaliser une discrimination par la fonction (révélation de la fonction résistance à l'acide clavulanique)
- de s'affranchir du bruit de fond (détection spécifique d'une population de germes au sein d'une population de germes similaires)
- de caractériser un échantillon : le premier test fonctionnel révèle que les échantillons 2 à 7 par exemple sont positifs, et le deuxième test fonctionnel permet de révéler qu'ils contiennent des germes polyrésistants.

De manière beaucoup plus classique il est envisageable de discriminer deux fonctions identiques (fonction protéase de HBV et fonction protéase de HIV) par une amplification spécifique du ou des gène(s) d'une de celles-ci (utilisation par exemple d'amorces de PCR spécifiques du gène de la protéase de HIV pour la détecter au sein d'un échantillon pouvant contenir aussi le gène de la protéase de HBV). Dans ce cas de figure le test fonctionnel n'est pas nécessairement discriminant.

### 2) Détection du virus HIV-1 par l'intermédiaire de sa fonction protéase.

La charge virale de HIV-1 est mesurée dans différents échantillons.

Les étapes a et b du procédé de l'invention ont été réalisées. La détection de l'activité (étape c)de la protéase a été réalisée en incubant à 37°C 10 µl de chacune de ces traductions (étape b) avec 60 µl de tampon (2M NaCl, 12 mM EDTA, 200 mM acétate de sodium, 2 mM DTT et 20% DMSO) et 10 µM final du peptide substrat BACHEM M1865 (peptide DABCYL-γ-Abu-Ser-Gln-Asn-Tyr-Pro-Ile-Val-Gln-EDANS) sur un volume final de 120 µl. Ce peptide FRET a pour propriété de libérer de la fluorescence lorsqu'il est clivé par la protéase du virus HIV. Un diagnostic positif se caractérise donc par l'apparition d'un signal fluorescent lorsqu'il est exposé aux UV.

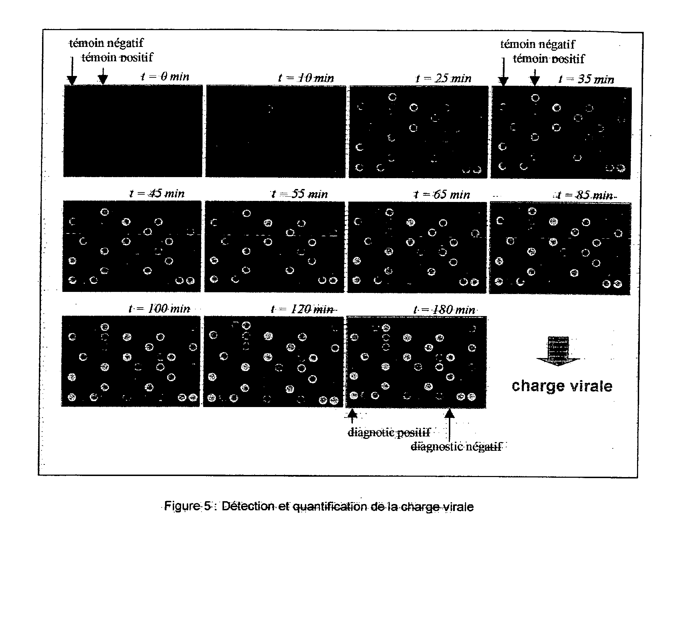

La figure 5 illustre les signaux obtenus pour les différents échantillons prélevés au cours du temps. Les intensités de fluorescence obtenues pour les différents signaux positifs correspondent aux différentes charges virales. En se référant à une gamme étalon, il est donc possible de quantifier le signal mesuré et d'en déduire la quantité de virus dans chaque échantillon.

Une fois qu'un échantillon est détecté comme étant positif, le diagnostic par le procédé de l'invention permet de caractériser la fonction détectée. La figure 5 illustre l'effet de concentrations croissantes d'un inhibiteur de la protéase HIV (inhibiteur non thérapeutique : la pepstatine A) sur l'activité de la protéase de l'échantillon.

Pour cela, 10 µl d'une traduction correspondant à un échantillon ont été incubés à 37°C avec 60 µl de tampon (2M NaCl, 12 mM EDTA, 200 mM acétate de sodium, 2 mM DTT et 20% DMSO),10 µM du peptide substrat BACHEM M1865 (peptide DABCYL-γ-Abu-Ser-Gln-Asn-Tyr-Pro-Ile-Val-Gln-EDANS) et 0 ou 500nM ou 1µM ou 10µM ou 30µM ou 60µM ou 80µM ou 100µM final de pepstatine A sur un volume final de 120 µl. La lecture a été réalisée par fluorimétrie à une longueur d'onde d'excitation de 340 nm et d'émission de 490 nm.

Une corrélation entre la diminution de l'activité et l'augmentation des concentrations croissantes en inhibiteur peut être observée.

En fixant un seuil de sensibilité (ou de résistance) (par exemple 50% de l'activité perdue pour une incubation de 30 minutes avec 50 mM d'inhibiteur), il est possible de déterminer si cette protéase est sensible ou non aux inhibiteurs de protéase existant, et d'adapter en direct une thérapie relative à l'échantillon prélevé.

Ceci démontre que la méthode de l'invention permet de détecter, quantifier et caractériser une fonction d'un organisme ou d'un processus à partir d'un seul échantillon.

Ces expériences peuvent être réalisées avec tout type de fonctions spécifiques d'organismes différents, ou d'un même organisme, ce qui permet alors de caractériser différentes cibles thérapeutiques (détection et caractérisation simultanées de la protéase et de la reverse transcriptase du virus HIV par exemple).

## Revendications

1. Méthode de détection d'une fonction connue, à partir des acides nucléiques présents dans un échantillon, **caractérisée en ce qu'**elle comprend les étapes suivantes :
- la préparation, à partir des acides nucléiques de l'échantillon, de molécules d'acides nucléiques comprenant le ou les gènes codant pour la ou les protéines correspondant à ladite fonction, et les éléments de contrôle nécessaires à la transcription et à la traduction du ou desdits gènes ;
- la transcription et la traduction *in vitro* des molécules d'acide nucléique préparée(s) à l'étape (a) ;
- la détection et/ou la mesure de la fonction correspondant à la ou aux protéines produites à l'étape (b).

2. Kit de diagnostic de la fonction d'une ou plusieurs protéine spécifique d'un organisme ou du développement d'une maladie ou d'une infection ou encore de cellules, dans un échantillon biologique, lesdites protéines étant produites dans un système acellulaire à partir des acides nucléiques de l'échantillon **caractérisé en ce qu'**il contient :
- une ARN polymérase,
- les quatre nucléotides triphosphates,
- un extrait de traduction,
- les réactifs pour la révélation de ladite fonction.

3. Un kit de diagnostic selon la revendication 2, **caractérisé en ce qu'**il contient en outre un témoin.

4. Un kit de diagnostic selon l'une des revendications 2 ou 3, **caractérisé en ce que** l'extrait de traduction est de la même origine ou d'une origine proche de celle de l'échantillon biologique diagnostiqué.

5. Un kit de diagnostic selon l'une quelconque des revendications 2 à 4, **caractérisé en ce qu'**il possède au moins un support pour la mise en oeuvre :
- de la transcription *in vitro,*
- de l'étape de traduction in vitro des transcrits,
- de révélation de ladite fonction.

6. Un kit de diagnostic selon l'une quelconque des revendications 2 à 5, **caractérisé en ce que** le kit possède des amorces non discriminantes pour la préparation, à partir des acides nucléiques de l'échantillon, de molécules d'acides nucléiques comprenant le ou les gènes codant pour une ou plusieurs protéines spécifiques dudit organisme ou développement d'une maladie ou d'une infection ou encore de cellules et **en ce que** l'on révèle lesdites fonctions par des tests fonctionnels permettant de différencier lesdites fonctions.

7. Un kit de diagnostic selon l'une quelconque des revendications 2 à 5, **caractérisé en ce que** le kit possède des amorces discriminantes pour la préparation, à partir des acides nucléiques de l'échantillon, de molécules d'acides nucléiques comprenant le ou les gènes codant pour une ou plusieurs protéines spécifiques dudit organisme ou développement d'une maladie ou d'une infection ou encore de cellules et **en ce que** l'on révèle lesdites fonctions par des tests fonctionnels permettant ou non de différencier lesdites fonctions.

8. Un support présentant une série d'emplacement pour la mise en oeuvre d'une méthode selon la revendication 1, ou pour être utilisé dans un kit selon l'une quelconque des revendications 2 à 5, **caractérisé en ce que** chacun desdits emplacements permet la détection et/ou la mesure d'une fonction différente.
